# EUROPEAN PATENT APPLICATION

(11) **EP 4 613 848 A1**
(43) Date of publication of application: **10.09.2025**
(21) Application number: 23885669.4
(22) Date of filing: 27.10.2023
(51) Int. Cl.: C12N 5/071, C12M 3/00

(54) **PLATE CONTAINING RENAL CELL AGGREGATES, AND PHARMACOKINETICS TEST KIT, TOXICITY TEST KIT, AND DRUG DISCOVERY AND DEVELOPMENT KIT USING SAME**

(30) Priority: 04.11.2022 JP 2022177566
(71) Applicant: Nikkiso Co., Ltd., Tokyo 150-6022 (JP)
(72) Inventor: TAKAHASHI, Etsushi, Kanazawa-shi, Ishikawa 920-0177 (JP)
(74) Representative: Hoffmann Eitle
(86) International application number: PCT/JP2023/038856
(87) International publication number: WO 2024/095913

(57) **Abstract**

Provided is a means for further improving evaluation accuracy in evaluating pharmacokinetics, toxicity, and drug efficacy using cell aggregates of a plurality of samples. A plate has a plurality of wells. A renal cell aggregate and a liquid are housed in each of the wells. Opening portions of the wells are covered with a covering member. A ratio of a total volume of the aggregate and the liquid to a total volume of the wells is a predetermined ratio.

## Description

### Technical Field

The present invention relates to a plate containing a renal cell aggregate, and a pharmacokinetic test kit, a toxicity test kit, and a drug discovery development kit using the plate.

### Background Art

A drug administered to a living body is absorbed into the living body, and then excreted from blood into urine through a proximal tubule in the kidney. Therefore, renal damage is often caused by nephrotoxicity of the drug. In drug discovery research, it is very important to examine pharmacokinetics in the kidney in order to discover an action of a drug. For this reason, development of a drug discovery support device capable of evaluating pharmacokinetics and toxicity using renal cells is desired. In addition, such a drug discovery support device is also useful for development of a therapeutic agent for a disease related to the kidney (for example, diabetes, kidney cancer, or hyperuricemia).

### Citation List

### Patent Literature

Patent Literature 1: JP 2021-191305 A

### Summary of Invention

### Technical Problem

Here, a cell aggregate of renal cells has been proposed as what is useful for confirmation of pharmacokinetics and drug discovery (for example, Patent Literature 1). Usually, pharmacokinetics, toxicity, and drug efficacy of a test target drug are confirmed and evaluated using cell aggregates of a plurality of samples (typically, a plate having a plurality of wells each of which includes a cell aggregate). An object of the present invention is to provide a means for further improving evaluation accuracy in evaluating pharmacokinetics, toxicity, and drug efficacy using such cell aggregates of a plurality of samples.

### Solution to Problem

An embodiment of the present invention is
a plate having a plurality of wells, in which
a renal cell aggregate and a liquid are housed in each of the wells,
opening portions of the wells are covered with a covering member (for example, a film or a lid), and
a ratio of a total volume of the aggregate and the liquid to a total volume of the wells is 35% or more and 75% or less.

In addition, an embodiment of the present invention may be the plate in which a specific gravity of the aggregate to the liquid is 1.00 or more and 1.20 or less.

In addition, an embodiment of the present invention may be the plate in which the covering member has a water vapor transmission rate of 500 g/m²/24h or more.

In addition, an embodiment of the present invention may be one in which the aggregate and the liquid are frozen.

In addition, an embodiment of the present invention is a pharmacokinetic test kit including the plate.

In addition, an embodiment of the present invention is a toxicity test kit including the plate.

In addition, an embodiment of the present invention is a drug discovery development kit including the plate.

### Advantageous Effects of Invention

According to the present invention, it is possible to provide a plate in which renal cell aggregates are housed in a plurality of wells, the plate being capable of further improving evaluation accuracy in evaluating pharmacokinetics, toxicity, and drug efficacy using cell aggregates of a plurality of samples.

### Brief Description of Drawings

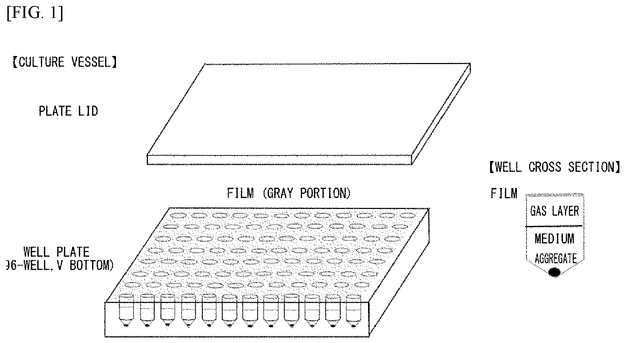

[Fig. 1] Fig. 1 is a schematic diagram of a plate according to the present embodiment.

### Description of Embodiments

### <<Plate containing renal cell aggregate>>

A plate according to the present embodiment has a plurality of wells. Each of the wells contains a liquid and one or more renal cell aggregates in the liquid. Furthermore, an opening portion of each of the plurality of wells is covered with a covering member. For example, Fig. 1 is a culture vessel including a well plate according to an example of the present embodiment. As illustrated on the left (upper and lower) of Fig. 1, the culture vessel includes a well plate (a 96-well plate with V bottom is exemplified in the drawing) whose surface is covered with a covering member (film in the drawing) and a well plate lid. In addition, as described later, as illustrated in the right of Fig. 1, a medium and an aggregate are housed in a well to such an extent that the well is not filled, and there is a gas layer between an upper surface of the medium and the covering member (film in the drawing). Hereinafter, each element will be described in detail.

### <Renal cell aggregate>

A renal cell used in the present embodiment may be any renal cell as long as it can be cultured, and a source thereof is not limited. The renal cell is preferably derived from a mammal, and is preferably derived from primates such as humans and monkeys. In addition, the renal cell may be derived from a normal kidney or a kidney having a disease depending on a purpose. Examples of the renal cell include cells constituting epithelium, cortex, proximal tubule, distal tubule, collecting duct, and glomerulus, and specific examples thereof include a renal_proximal tubule epithelial cell (RPTEC), and a mesangial cell. The renal cell may be a primary cell or a renal cell derived from a stem cell such as an iPS cell or an ES cell. In addition, the renal cell may be an immortalized renal cell, an established cell (an HK-2 cell or the like), a cell derived from other animal species (an MDCK cell, an LLC-PK1 cell, a JTC-12 cell, or the like), or a forcibly expressed cell obtained by introducing a gene into a renal cell in order to express a protein such as a specific transporter. More specific examples of the renal cell include a human proximal tubule epithelial cell, a human distal tubule epithelial cell, and a human collecting duct epithelial cell collected and isolated from the kidney, and a proximal tubule epithelial cell, a distal tubule epithelial cell, and a collecting duct epithelial cell induced to differentiate from a human iPS cell or a human ES cell. For use in drug discovery research, a renal proximal tubule epithelial cell is preferable, and a renal proximal tubule epithelial cell derived from a human normal kidney is particularly preferable.

Here, the "aggregate" of cells refers to a massive aggregate of several or more cells. The aggregate is also referred to as an aggregated mass or a spheroid. The number of cells constituting the aggregate is, for example, 5 or more, 25 or more, 50 or more, preferably 100 or more, more preferably 125 or more, still more preferably 200, and particularly preferably 500 or more. The number of cells constituting the aggregate is, for example, 4000 or less, preferably 10,000 or less, and more preferably 2000 or less, or 1000 or less. When the number of cells constituting the aggregate is within such a range, almost all the cells maintain survival in a culture solution, and higher homogeneity among the plurality of aggregates in the plate is possible.

### (Form parameter)

Examples of a form parameter include the diameter, volume, cross-sectional area, circumferential length, compactness, roundness, and aspect ratio of the aggregate. Hereinafter, each of the parameters will be described in detail.

The diameter of the aggregate is preferably, for example, 100 µm or more and 800 µm or less. Note that the diameter of the aggregate is defined as a maximum width of the aggregate. That is, the diameter of the aggregate is the length of the longest one of straight lines connecting two points on an outer edge of the aggregate. The diameter of the aggregate can be measured using, for example, a photograph taken with a phase contrast microscope on the basis of a well-known method. BZ-X710 (Keyence Corporation) can be used as the phase contrast microscope, and analysis software can be used to measure the diameter.

The volume of the aggregate is preferably, for example, 0.001 mm³ or more and 0.300 mm³ or less. The volume of the aggregate can be calculated from a measured diameter because the aggregate is substantially spherical.

The cross-sectional area of the aggregates is preferably 40,000 µm² or more and 100,000 µm² or less, and more preferably 45000 µm² or more and 90,000 µm² or less. Here, the cross-sectional area (also a circumferential length, compactness, roundness, and aspect ratio described below) is calculated by recognizing one aggregate in an image {slice image (cross-sectional view)} with a CQ1 (confocal image cytometer, manufactured by Yokogawa Electric Corporation) on the basis of a well-known method.

The circumferential length of the aggregates is preferably 600 µm or more and 1800 µm or less, and more preferably 800 µm or more and 1600 µm or less.

The compactness of the aggregate is preferably 1.0 or more and 3.0 or less, and more preferably 1.2 or more and 2.5 or less.

The roundness of the aggregate is preferably 0.3 or more and 1.0 or less, and more preferably 0.4 or more and 1.0 or less.

The aspect ratio of the aggregate is preferably 1.0 or more and 2.0 or less, and more preferably 1.0 or more and 1.5 or less.

### (Variation coefficient of ATP)

A variation coefficient of ATP in a plurality of cell aggregates is preferably 20% or less, more preferably 15% or less, and most preferably 10% or less. A lower limit value thereof is 0%.

### (Variation coefficient of form parameter)

A variation coefficient of the cross-sectional area in the plurality of cell aggregates is preferably 20% or less, more preferably 15% or less, and still more preferably 10% or less. A variation coefficient of the circumferential length in the plurality of cell aggregates is preferably 20% or less, more preferably 15% or less, and still more preferably 10% or less. A variation coefficient of the compactness in the plurality of cell aggregates is preferably 25% or less, more preferably 20% or less, and still more preferably 15% or less. A variation coefficient of the roundness in the plurality of cell aggregates is preferably 25% or less, more preferably 20% or less, and still more preferably 15% or less. A variation coefficient of the aspect ratio in the plurality of cell aggregates is preferably 20% or less, more preferably 15% or less, and still more preferably 10% or less.

### <Plate>

The plate containing a renal cell aggregate group is not particularly limited as long as it has a plurality of wells capable of housing one or more aggregates in one well, and any plate can be used. Examples of the culture vessel include types such as a 6-well plate, a 24-well plate, a 48-well plate, a 96-well plate, a 384-well plate, and dishes of various sizes. In addition, the shape of a bottom is not particularly limited, and examples thereof include a flat bottom, a V bottom, and a U bottom.

### <Liquid>

The liquid present in each well is not particularly limited, and may be a medium when an aggregate is prepared, a fresh medium, or a liquid other than the medium (for example, physiological saline or a buffer solution). The liquid may contain salts, a buffer, serum, a vitamin, an amino acid, glucose (sugar), an electrolyte, an antibiotic, and a growth factor (a compound or a protein).

### <Covering member>

A preferable covering member (for example, a film such as a sheet-shaped film) has a water vapor transmission rate (measured under conditions of a temperature of 40°C and a humidity of 90%RH in accordance with JIS Z0208 method) of preferably 500 g/m²/24 hr or more, more preferably 2000 g/m²/24 hr or more, most preferably 4000 g/m²/24 hr or more. Note that an upper limit value of the water vapor transmission rate is, for example, 100,000 g/m²/24 hr or less, 50,000 g/m²/24 hr or less, 25000 g/m²/24 hr or less, or 10,000 g/m²/24 hr or less. The preferable covering member has a pore size of 10 µm to 50 µm. Note that this covering member may cover all the wells in the plate with one sheet, may cover each well group after dividing the wells into a plurality of well groups, or may cover the wells for each well. Note that, when the covering member is not flat but has, for example, an uneven shape on the basis of a plane of the opening portion, the "volume of the well" in the present specification and claims is a volume in consideration of the uneven shape (that is, when the covering member has a recessed shape, a "recessed volume" is subtracted from the "volume of the well" on the basis of the plane of the opening portion, and when the covering member has a protruding shape, a "protruding volume" is added to the "volume of the well" on the basis of the plane of the opening portion).

### <Volume ratio of aggregate to liquid>

A volume ratio of the aggregate to the liquid in one well is preferably 0.001% or more, 0.002% or more, or 0.003% or more, and preferably 0.800% or less, 0.500% or less, or 0.200% or less.

### <Volume ratio of content (liquid + aggregate) to volume of well>

A volume ratio of the content (liquid + aggregate) to the volume of the well is preferably 35% or more, 37.5% or more, or 40% or more, and preferably 75% or less, 70% or less, or 60% or less. Note that the volume of the aggregate here is the total volume of a plurality of aggregates when the plurality of aggregates is present in the well. Here, a reason why the volume ratio is more preferably equal to or more than the above-described lower limit value will be described. First, the amount of dissolved oxygen per cell increases, and the cells can be maintained more stably. In addition, by reducing a concentration of cell waste products, it is possible to reduce a damaging influence on the cells. Furthermore, a risk of aspirating the aggregate at the time of medium replacement is further reduced, and high uniformity can be maintained in the culture plate. In addition, a situation in which cell culture cannot be maintained by drying of the medium can be further avoided. Meanwhile, a reason why the volume ratio is more preferably equal to or less than the upper limit value will be described. First, supply of air (oxygen) to the medium in the well can be ensured, and therefore the amount of dissolved oxygen increases, and deterioration of a function of the cells can be prevented. In addition, a gas layer is formed between the aggregate and the liquid medium in the well, and therefore appropriate air (oxygen) can be supplied to the medium through an air-permeable sheet. Furthermore, since the gas layer is formed, the aggregate does not adhere to the sheet even when vibration due to transportation or the like is assumed, a loss of the aggregate can be effectively prevented, and a culture plate in which aggregates are more uniformly arranged can be obtained.

For example, the well volume of a 96-well plate is generally 280 to 310 mm³ (for example, about 310 mm³ for PrimeSurface 96 V manufactured by Sumitomo Bakelite Co., Ltd., and about 280 mm³ for EZ BindShut SP 96-well plate manufactured by AGC Techno Glass Co., Ltd.). At this time, the volume of the content (cells and liquid medium) is preferably 120 to 200 mm³, and more preferably 130 to 160 mm³.

### <Specific gravity of aggregate to liquid>

A lower limit value of the specific gravity of the aggregate to the liquid in the well is preferably 1.00 or more, and preferably 1.20 or less, 1.15 or less, or 1.10 or less. With such a specific gravity, the aggregate does not float on a liquid surface and is not excessively pressed to a bottom by its own weight, and therefore the aggregate is less likely to be affected by a change in extracellular environment (particularly during transportation). This results in higher homogeneity between the plurality of aggregates in the plate.

### <Number in well>

The number of aggregates in the well is one or more. Here, in a case of a plurality of cells, 2 or more and 1000 or less cells are preferable. For example, in a case of a 6-well plate, an aspect is assumed in which 500 to 1000 cells/well are arranged.

### <<Method for producing cell aggregate>>

By using a medium and a culture vessel suitable for cells to be cultured, renal cells can be cultured according to a conventional method, for example, under conditions of 37°C and 5%CO₂. The culture may be any of static culture, shaking culture, stirring culture, and the like. The culture may be adhesion culture, but it is preferable to perform the culture in a non-adhesion state (for example, suspension culture) for at least a part of a period. The renal cells can be formed into an aggregate by being cultured in a non-adhesion state in a culture vessel. The "non-adhesion state" refers to a state in which all or most of the cells are not adhering to a surface of the culture vessel, and includes a state in which all or most of the cells are separated from the surface of the culture vessel and a state in which even when the cells are in contact with the surface of the culture vessel, the cells can be easily separated from the surface of the culture vessel by coating of the culture vessel, convection of the medium, or the like without using a tool, an enzyme, or the like.

For example, in some cases, a renal cell aggregate is formed within 24 hours after initiation of culture of the renal cells. Then, by culturing the renal cells in an aggregate state for a part of a period, it is possible to restore a physiological function of the renal cells, the physiological function having been lowered by dedifferentiation. A period during which the renal cells are cultured in a state of not adhering to the culture vessel is generally desirably 120 hours or more. This makes it possible to obtain cultured renal cells in a state of higher expression of the physiological function. During the culture period, the medium is preferably replaced periodically. For example, the medium is replaced every two days.

As the medium, any known medium can be appropriately used. For example, in a case of culture of renal proximal tubule epithelial cells, a commercially available tubular cell medium can be used, and preferable examples thereof include REGM (registered trademark) (LONZA), EpiCM (registered trademark) (ScienCell), and KeratinocyteSFM (registered trademark) (Thermo Fisher Scientific).

In addition, conventionally known materials and additives useful for cell culture can be appropriately used. For example, collagen I (type I collagen) can be added to the medium. Collagen I has an action of causing the renal cells to adhere to each other. Therefore, formation of an aggregate is promoted by culturing the renal cells in a medium containing collagen I. Collagen I is preferably full-length collagen I, but may be an α1 chain or an α2 chain constituting collagen I, or a collagen peptide obtained by fragmenting chains. Ae source of collagen I is not particularly limited, and collagen-I may be derived from humans or other animals.

Any culture vessel can be used. Here, in order to promote formation of an aggregate, the culture vessel is preferably subjected to a cell non-(low) adhesion treatment or is preferably made of a cell non-(low) adhesion material. Examples of the cell non-(low) adhesion treatment include a cell non-adhesion hydrogel coating treatment, a 2-methacryloyloxyethyl phosphorylcholine (MPC) coating treatment, a ProteoSave (registered trademark) SS coating treatment, and a mirror polishing treatment to a surface of the vessel. Examples of the cell non-(low) adhesion material include glass, and polymer materials such as a low-density polyethylene, a medium-density polyethylene, polyvinyl chloride, a polyethylene-vinyl acetate copolymer, a poly(ethylene-ethyl acrylate) copolymer, a poly(ethylene-methacrylate) copolymer, a poly(ethylene-vinyl acetate) copolymer, and mixtures of two or more of these polymers. An aggregate cultured by the cell non-(low) adhesion treatment is transferred to another culture vessel to be used. In this case, a culture vessel subjected to the cell non-(low) adhesion treatment or a cell adhesion treatment can be used. The shape of a well bottom of the culture vessel to which the aggregate has been transferred is not particularly limited, and examples thereof include a flat bottom, a V bottom, and a U bottom.

When a large amount of aggregates are formed, a high-density spheroid preparing plate or a dish can be used. Furthermore, a culture vessel such as a spinner flask may be used as necessary. For example, it is preferable to use a culture vessel of ELPLASIA (registered trademark) series (Corning), a culture vessel of EZSPHERE (registered trademark) series (AGC Techno Glass Co., Ltd.), or the like. Examples of the culture vessel include types such as a 6-well plate, a 24-well plate, a 96-well plate, a 384-well plate, and dishes of various sizes, and the number of aggregates that can be prepared varies depending on the size of a bottom area of the vessel. For example, when a 96-well plate (V bottom), a 96-well plate (U bottom), or a 384-well plate (U bottom) subjected to a low adhesion treatment is used, one aggregate is formed in one well.

An aggregate prepared using a high-density spheroid preparing plate, a dish, or the like can be collected and cultured while being suspension-shaken. In a case where an aggregate is cultured while being suspension-shaken, it is preferable to culture the aggregate by placing a dish, a plate, or the like subjected to a cell non-(low) adhesion treatment on a shaker. As the shaker, a reciprocating shaker and a swirling shaker can be used.

### <<Storage and transportation method>>

A method for storing and transporting the plate according to the present embodiment includes a maintenance step of controlling the plate containing a renal cell aggregate to a temperature (hereinafter, also referred to as a "maintenance temperature" for convenience) that is lower than a culture temperature of the renal cells and at which the liquid does not freeze when the renal cells are stored or transported. Adopting this step enables higher homogeneity among the plurality of aggregates in the plate.

As the temperature that is lower than the culture temperature of the renal cells and at which the liquid does not freeze, an optimum temperature can be appropriately selected depending on, for example, the type and use of the cells and/or a period required for storage or transportation. Note that the culture temperature is a temperature suitable for survival of cells, and the cells may proliferate or do not have to proliferate. Since the culture temperature of the cells is typically 37°C, the maintenance temperature is desirably lower than 37°C. On the other hand, when the maintenance temperature is too low, partial freezing occurs in the cells, and thus a cell survival ratio in the aggregate may be reduced. Therefore, the maintenance temperature is desirably 0°C or higher. The maintenance temperature can be, for example, 0°C or higher and lower than 37°C. In a case where it is desirable to maintain the temperature at a relatively high temperature, the maintenance temperature is preferably 10°C or higher and lower than 37°C, and more preferably 20°C or higher and lower than 28°C. In a case where it is desirable to maintain the temperature at a relatively low temperature, the maintenance temperature is preferably 0°C or higher and lower than 10°C, and more preferably 3°C or higher and lower than 8°C. Note that, in the present specification, for convenience, a temperature range of 10°C or higher and lower than 37°C may be referred to as "normal temperature", and a temperature range of 0°C or higher and lower than 10°C may be referred to as "refrigeration".

In a case of storage, an incubator, a thermostatic chamber, a refrigerator, or the like that can be adjusted to a desired temperature can be used in the maintenance step.

In the case of transportation, it is preferable to use a heat-insulating container such as expanded polystyrene in the maintenance step. The size of the heat-insulating container is not particularly limited as long as a container for storage and transportation can be packed therein. When there is a gap between the heat-insulating container and the container for storage and transportation, a cushioning material is preferably packed therein such that the container for storage and transportation does not move. As the heat-insulating container, a paper container or a cardboard container can also be used.

Note that the maintenance step is not necessarily performed by standing. Therefore, the maintenance step can be performed under a situation where there is vibration to such an extent that the aggregate is not broken, such as on a shaker or during transportation.

### <<Freezing method>>

One aspect of the present embodiment may be a plate containing a renal cell aggregate in a frozen state. In this case, a method for producing the plate in a frozen state includes, for example, a step of producing a renal cell aggregate, and then freezing the renal cell aggregate while substantially maintaining an aggregated state of the aggregate, a step of putting the frozen aggregate and a liquid (preferably, a cryopreservation medium described in detail below) into a well, and a step of freezing the liquid in the well. Hereinafter, the step of freezing the aggregate will be described in detail.

The aggregate is frozen by cooling the aggregate under a low temperature at which the cells can freeze until the cells freeze. Here, it is preferable to freeze the renal cells while adjusting a temperature drop rate of the cells to be frozen to fall within a predetermined range. The temperature drop rate can be adjusted by using a commercially available cryopreservation container, a program freezer capable of setting freezing conditions of cells and tissues, or the like. The temperature drop rate of the cells during freezing can be set with as little damage to the cells during freezing as possible. For example, in a case of slow freezing, the temperature drop rate can be within a range of about 0.2°C to about 3°C per minute, and a rate of about 1°C per minute is preferable.

The renal cells can be cooled using a freezer or liquid nitrogen as a general freezing method. For example, when an aggregate of renal proximal tubule epithelial cells is frozen, it is preferable to use an ultra-low temperature freezer that can be set to a temperature of -80°C. When the aggregate is stored in a liquid nitrogen storage container, the aggregate can be stored at a temperature of -196°C in a liquid phase, and can be stored at a temperature of -150°C to -196°C in a gas layer.

"Substantially maintaining an aggregated state of the aggregate" in the freezing step means that the form of the aggregate is not impaired without performing an operation (for example, trypsinization) of intentionally breaking or dispersing the aggregate contained in the collected renal cells. In addition, "substantially" means that a decrease in the amount of the aggregate after freezing is within a range that does not cause a problem as compared with the amount of the aggregate contained in the collected renal cells before freezing, and does not necessarily mean that the cells constituting the aggregate are not dispersed at all during freezing.

Note that any step, for example, another step useful for the cryopreservation of cells, may be included between the collection step and the freezing step as long as it does not have a significant adverse effect on maintenance of the form of the aggregate. For example, a step of adding a preferable cryopreservation medium to the collected renal cells can be included.

Examples of the cryopreservation medium include a medium containing a cryoprotectant component that reduces damage caused by intracellular ice crystals, for example, any of dimethyl sulfoxide (5 to 15%), serum derived from mammals, dextran, glycogen, methyl cellulose or carboxymethyl cellulose, polyethylene glycol, polyvinyl pyrrolidone, glucose, and sucrose. The medium can be a liquid such as a medium. A solution in which two or more of these cryoprotectants are appropriately blended is preferable. Therefore, as a commercially available cryopreservation medium, a commercially available cryopreservation solution such as CELLBANKER (registered trademark) 1plus (Zenoaq Resource Co., Ltd.), CELLBANKER (registered trademark) 1 (Zenoaq Resource Co., Ltd.), or CELLBANKER (registered trademark) 2 (Zenoaq Resource Co., Ltd.) may be used.

### <<Application>>

The plate housing the cell aggregate according to the present embodiment therein can be provided as a drug evaluation system or a cell product. Examples of the drug evaluation system include a system for evaluating pharmacokinetics in renal cells and nephrotoxicity. In addition, such renal cells can also be used as a tool for mechanism analysis of diseases related to the kidney, such as diabetes, kidney cancer, and hyperuricemia, and therapeutic agent search.

The present invention is not limited to the above-described embodiments, and various modifications such as design changes can be made on the basis of knowledge of those skilled in the art, and embodiments to which such modifications are made are also included in the scope of the present invention.

### Examples

### <Method for producing cell aggregate>

Human renal proximal tubule epithelial cells {Clonetics (registered trademark), Catalog No. CC-2553, RPTEC-kidney proximal tubule epithelial cells} obtained from LONZA were used as renal cells. A cryovial stored in a liquid nitrogen storage was immersed in a thermostat at 37°C for thawing. After thawing, a cell suspension in the cryovial was mixed with a recommended medium {REGM (registered trademark), LONZA} and cultured in a culture dish. The cells were cultured under conditions of 37°C and 5%CO₂ while the medium was replaced at a frequency of once every two days. The cells were collected before becoming confluent, and seeded on a 96-well V-bottom plate {PrimeSurface (registered trademark) plate 96 V, Sumitomo Bakelite Co., Ltd.} subjected to a cell low adhesion treatment such that the number of cells was 1000 per well, and cultured to form an aggregate. The aggregate was cultured while the medium was replaced at a frequency of once every two days. Noted that "confluent" means that a ratio of an area occupied by the cells to the entire culture surface of the culture vessel is about 100%, that is, means a state in which the cells have proliferated to the full extent of the culture surface without gaps. After the cells were cultured for 240 hours or more, a surface of the plate containing the culture solution containing the aggregate was covered with a film {Aera Seal (Excel Scientific)/water vapor transmission rate: 4200 g/m²/24 hr; pore size: 10 µm to 50 µm}. At this time, the specific gravity of the cell aggregate to the medium was 1.00 to 1.07. A volume ratio of the content (liquid + aggregate) to the volume of the well was 48%. Furthermore, a volume ratio of the aggregate to the liquid in the well was 0.013%. Thereafter, the plate was placed in a container made of expanded polystyrene, and allowed to stand in a room (air conditioning set temperature: 25°C) for 72 hours.

### <Amount of ATP>

For the cell aggregate groups (96 groups) after being allowed to stand for 72 hours, the amount of ATP was measured by a luminescence method using CellTiter-Glo (registered trademark) 3D Cell Viability Assay (Promega Corporation). Specifically, the aggregates were collected together with the medium, and CellTiter-Glo 3D Reagent was added thereto in an amount equal to the volume of the medium. The mixture was incubated at room temperature for 30 minutes. The mixture was mixed well, and then a luminescence value was measured with a microplate reader (Perkin Elmer).

### <Measurement of diameter, volume, and surface area of aggregate>

For the cell aggregate groups (96 groups) after being allowed to stand for 72 hours, a state of each aggregate in the well was observed with a phase contrast microscope BZ-X710 (Keyence Corporation) and imaged. The diameter was measured from a form photograph using analysis software (Keyence). Furthermore, the volume was calculated from the obtained diameter, and a volume ratio of the aggregate to the medium was calculated. In addition, the cross-sectional area, circumferential length, compactness, roundness, and aspect ratio were calculated by recognizing one aggregate in an image {slice image (cross-sectional view)} with a CQ1 (confocal image cytometer, manufactured by Yokogawa Electric Corporation) on the basis of a well-known method.

Results thereof are presented in Tables. Table 1 presents measured values and variation coefficients of various form parameters. Table 2 presents a measured value and a variation coefficient of ATP per aggregate. As can be seen from these Tables, it was confirmed that variations in form parameters and ATP among a plurality of aggregates were suppressed even after a lapse of a long period of time. Note that a frozen plate containing a frozen aggregate produced by the same method as in Example was also subjected to the same evaluation after being thawed, and similar results were obtained.

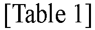

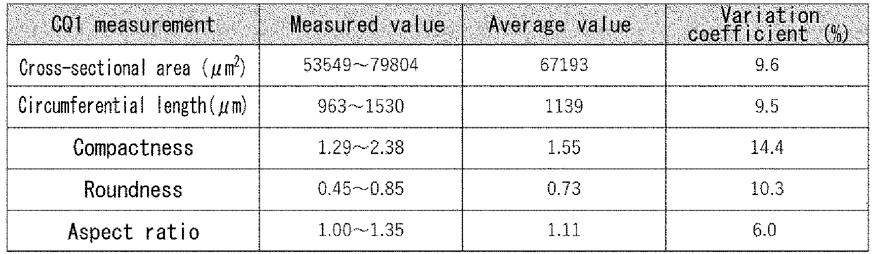

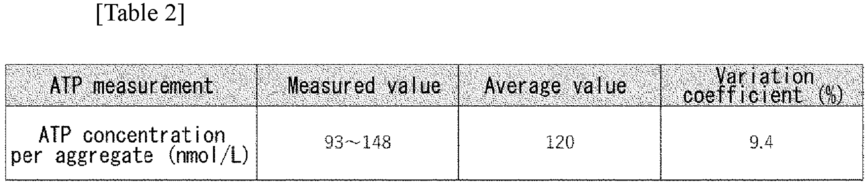

### Industrial Applicability

The present invention relates to a plate containing a renal cell aggregate, and a pharmacokinetic test kit, a toxicity test kit, and a drug discovery development kit using the plate, and can be applied to, for example, an industry handling therapeutic drug development for a disease related to the kidney.

## Claims

1. A plate comprising a plurality of wells, wherein
a renal cell aggregate and a liquid are housed in each of the wells,
opening portions of the wells are covered with a covering member, and
a ratio of a total volume of the aggregate and the liquid to a total volume of the wells is 35% or more and 75% or less.

2. The plate according to claim 1, wherein a specific gravity of the aggregate to the liquid is 1.00 or more and 1.20 or less.

3. The plate according to claim 1, wherein the covering member has a water vapor transmission rate of 500 g/m²/24 hr or more.

4. The plate according to claim 1, wherein the aggregate and the liquid are frozen.

5. A pharmacokinetic test kit comprising the plate according to any one of claims 1 to 4.

6. A toxicity test kit comprising the plate according to any one of claims 1 to 4.

7. A drug discovery development kit comprising the plate according to any one of claims 1 to 4.
